# EUROPEAN PATENT SPECIFICATION

(11) **EP 3 376 965 B1**
(45) Date of publication and mention of the grant of the patent: **25.03.2020**
(21) Application number: 17713089.5
(22) Date of filing: 10.02.2017
(51) Int. Cl.: A61B 17/02

(54) **A RETRACTOR USED AT OPEN HEART SURGERIES**
SPREIZER FÜR OPERATIONEN AM OFFENEN HERZEN
RÉTRACTEUR UTILISÉ POUR DES CHIRURGIES À COEUR OUVERT

(43) Date of publication of application: 26.09.2018
(73) Proprietor: Tüysüz, Mehmet Erin, 33110 Yenisehir Mersin (TR)
(72) Inventor: Tüysüz, Mehmet Erin, 33110 Yenisehir Mersin (TR)
(86) International application number: PCT/TR2017/050060
(87) International publication number: WO 2018/147820

(56) References cited:
- WO-A2-99/15069
- DE-U1-202011 051 999
- FR-A1- 2 807 313
- FR-A1- 2 807 313
- US-A- 4 813 401
- US-A1- 2008 147 109

## Description

### Technical Field

Invention is an retractor which is used at open heart surgeries to make the mitral and tricuspid valves apparent during the surgical operation.

### Prior Art

Mitral valve is between the left atrium and the left ventricle of the heart. It has two leaflets and these leaflets are attached to the two papillary muscles (muscle bulge) by means of the bonds named as "chorda". A large number of veins and transmission lines pass around the annulus.

Seeing of the valves better during the mitral valve surgery, is one of the leading conditions to repair or change. In order to evaluate the medical condition better, the valves and the tissues under the valves have to be seen by pulling with the retractors without causing any valve deformation.

The main object of the mitral valve surgery is to protect the natural valve of the patient. If the protection of the valve can not be provided, the valve is changed. In order to repair or change the mitral valve better, the valve and the other tissues have to be seen without a strong retractor pulling. To repair the mitral valve better, the structures of the leaflets, comissures, tissues under the valve, chordas and papillary muscles have to be evaluated magnificently. The strong pulling during the repair, can cause the location changes of the valve structure and this can cause the wrong repairments.

Nowadays an auxiliary staff is needed at the mitral valve surgery. To make the mitral and tricuspid valves apparent, is provided with the aid of an auxiliary staff. However this case makes difficult the operation and the efficiency of the operation is decreased.

The patent search has been made related to the prior art, US6312447 numbered patent application has been found. This application discloses a repair device used at mitral valve surgery.

FR2807313A1 relates to a soft tissue retractor assembly in orthopedics, in particular the spine, intended to prepare the placement of spinal implants, in particular in the cervical spine by anterior approach.

US4813401A relates to a surgical retractor for adjustable mounting on a supported holder includes an elongated stem of cylindrical cross-sectional configuration and blade means attached to one end of the stem, the stem being optionally provided with a plurality of spaced circumferential first means for selective engagement by adjustable complemental second means for restraining longitudinal movement of the stem in one direction, and optionally including an inner section hingedly connected to an outer section, providing a handle for pulling or rotating the retractor.

US2008/147109A1 relates to an instrument for endoscopic surgery comprises a retractor having independently spreadable retractor blades mounted at the distal end of a tube, through which a balloon dissector can be passed. The balloon is transparent, and the balloon dissector is provided with an image transmitter for visualization of tissue dissection.

### Explanation of the Invention

The object of the invention is to make the valve apparent during the surgical operation in a way that the retractor is placed into the heart chamber of the heart valve fleetingly, without any auxiliary staff during the open heart surgery.

Another object of the invention is to simplify the valve repairment or replacement the valve with the new one. Thanks to the retractor, the valve surgeries are realized without second surgeon.

Another object of the invention is to decrease the number of the required surgical incisions during the minimal invasive heart surgeries than to the currently used retractors.

Another object of the invention is that the retractor can have different diameters and it can be adjusted according to the atrium diameter of different hearts.

### Figures of the Invention

Figure-1: The general perspective view of the retractor of the subject invention.
Figure-2: The upper view of the retractor of the subject invention.
Figure-3: The close-up general montage perspective view of the retractor of the subject invention.
Figure-4: The demounted perspective view of the retractor of the subject invention.

### Reference Numbers

100-Retractor
110-Outer tube
111-Motion channel
112-Concave surface
120-Inner tube
121-Adjustment holes
122-Holders
122.1-Connection rings
123-Pins
124- Shrinker areas
125-Extensive areas
130-Tissue pushers
131-Slings
132-Sling holder
133-Axial motion bearings
134-Movable pushers
135- Intermediate ribs
136-Tissue pusher end
E- Vision gap
P- Ellipse form

### Detailed Description of the Invention

The present invention is disclosed in the appended set of claims. The general montage perspective view of a retractor (100) which is used at open heart surgeries to make the mitral and tricuspid valves apparent during the surgical operation, is given in Figure-1. retractor (100) is placed by intraatrial. After the cutting of the chamber of the heart (atriotomy), it is placed into the heart in a closed manner. It provides an ellipse space by means of the parts which move telescopically. It pushes the upper wall of the heart chamber by its spoons, it brings the valves to the proper vision area for the surgical operation.

The retractor (100) generally comprises an outer tube (110), an inner tube (120) placed to the inner part of the outer tube (110) wherein said inner tube (110) has a smaller diameter than the diameter of said outer tube (120), a great number of tissue pushers (130) which are constituted in a manner that they can move along the outer tube (110). Said outer tube (110) includes at least one motion channel (111) formed on its concave surface (112). The inner tube (120) includes a great number of adjustment holes (121) formed on its concave surface. Further that, it comprises a great number of sling holders (132) which are hanged to the motion channel (111) by the slings (131). On the other hand, it comprises movable pushers (134) rotated in x-axis by the sling holders (132) and comprises axial motion bearings (133) providing the rotational motion between the movable pushers (134) and the sling holders (132).

Preferably three tissue pushers (130) are used in the system. Optionally, the number of the tissue pushers (130) can be changed. It comprises tissue pusher ends (136), which push the atrium wall of the heart upwardly and makes the heart valve apparent, allied with the motion of the movable pushers (134) of the tissue pushers (130). The tissue pusher ends are formed with radius.

It further comprises a great number of intermediate ribs (135) placed in a parallel manner to each other, which increase the strength of the movable pushers (134) during the pushing motion. These intermediate ribs (135) are the additional elements which provide high resistance to the movable pushers (134) and to the system.

Further that, the retractor comprises holders (122) fixed to the ends of the outer tube (110) by the connection rings (122.1), said holders (122) with a half-crescent form, are attached to the adjustment holes (121) of the said inner tube (120) by the pins (123).

The inner tube (120) used in the system, have a half-crescent hollow form, said inner tube's ends have shrinker areas (124) wherein said ends are engaged into the outer tube (110), on the other hand said inner tube has extensive areas (125) towards its middle portion. The retractor comprises an outer tube (110) and an inner tube (120) which form an ellipse form (P) to have a vision gap (E) when they are engaged to each other. Said holders (122) have pins (123) which penetrate into the adjustment holes (121). The holders (122) limit the motion to fix the diameter when the proper space is provided. They are formed on each ends of the outer tube (110). They are opened with a 90 degree angle towards the center of the ellipse.

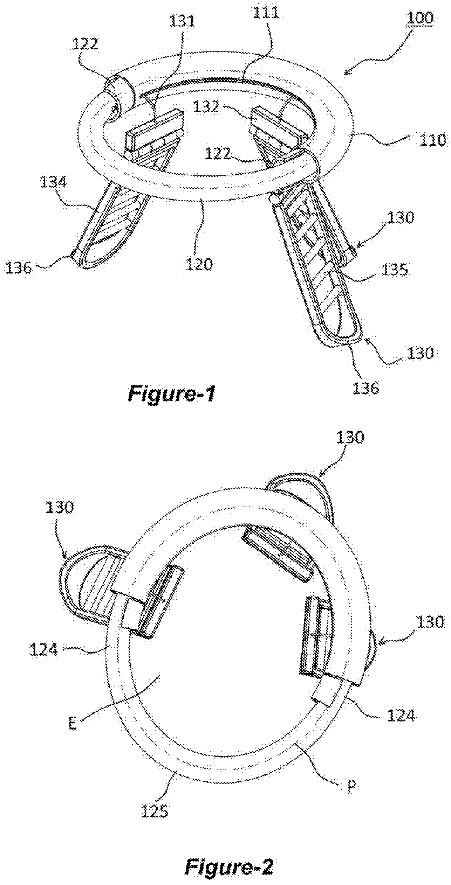

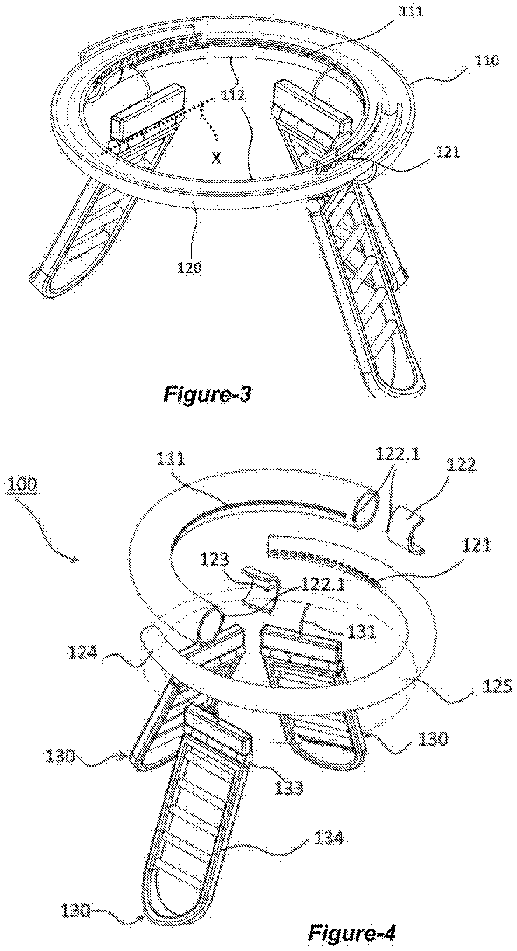

## Claims

1. A retractor (100) comprising an outer tube (110), in the form of a torus segment, with a concave surface (112) that faces towards the center of a surrounded space, said concave surface comprising at least one motion channel (111) formed on said concave surface, an inner tube (120) in the form of a torus-segment movably and telescopically placed in an inner part of the outer tube (110), such that said inner tube (120) and said outer tube (110) define said surrounded space, a plurality of tissue pushers (130) hung to the motion channel (111) and which are constituted in a manner that they can move along the outer tube (110), said retractor being adapted to be used at open heart surgeries to make the mitral and tricuspid valves apparent during the surgical operation, said retractor being **characterized in that** it further comprises:
- a plurality of adjustment holes (121) formed on said surface of the inner tube (120) that faces toward the center of the surrounded space,
- holders (122) fixed to each end of the outer tube (110) by connection rings (122.1), wherein said holders (122) are attached to the adjustment holes (121) of said inner tube (120) by pins (123).

2. -A retractor (100) according to Claim 1, **characterized in that** said tissue pushers (130) comprise a plurality of sling holders (132), which are hung to the motion channel by slings (131).

3. A retractor (100) according to Claim 2, **characterized in that** said tissue pushers (130) comprise movable pushers (134) which are rotated around an axis of the sling holders (132).

4. A retractor (100) according to Claim 3, **characterized in that** said tissue pushers (130) comprise axial motion bearings (133) providing the rotational motion between the movable pushers (134) and the sling holders (132).

5. A retractor (100) according to Claim 3, **characterized in that** said tissue pushers (130) comprise a tissue pusher end (136), which is configured to push the atrium wall of the heart upwardly and makes the heart valve apparent, allied with the motion of the movable pushers (134).

6. A retractor (100) according to Claim 4, **characterized in that** said tissue pushers (130) comprise intermediate ribs (135) placed in a parallel manner to each other, which increase the strength of the movable pushers (134) during a pushing motion.

## Patentansprüche

1. Retraktor (100) mit einem Außenrohr (110) in Form eines Torussegments mit einer konkaven Oberfläche (112), die zur Mitte eines umgebenen Raums weist, wobei die konkave Oberfläche mindestens einen Bewegungskanal (111) auf der konkaven Oberfläche ist ein Innenrohr (120) in Form eines Torussegments beweglich und teleskopisch in einem Innenteil des Außenrohrs (110) angeordnet, so dass das Innenrohr (120) und das Außenrohr (110) definieren den umgebenen Raum eine Vielzahl von Gewebeschiebern (130), die an dem Bewegungskanal (111) aufgehängt sind und die so aufgebaut sind, dass sie sich entlang des Außenrohrs (110) bewegen können, wobei der Retraktor dafür ausgelegt ist, verwendet zu werden Operationen am offenen Herzen, um die Mitral- und Trikuspidalklappen während des chirurgischen Eingriffs sichtbar zu machen, wobei der Retraktor **dadurch gekennzeichnet ist, dass** er ferner umfasst:
- eine Vielzahl von Einstelllöchern (121), die an der Oberfläche des Innenrohrs (120) ausgebildet sind, die der Mitte des umgebenen Raums zugewandt ist,
- Halter (122), die an jedem Ende des Außenrohrs (110) durch Verbindungsringe (122.1) befestigt sind, wobei die Halter (122) durch Stifte (123 an den Einstelllöchern (121) des Innenrohrs (120) befestigt sind).

2. Retraktor (100) nach Anspruch 1, **dadurch gekennzeichnet, dass** die Gewebeschieber (130) eine Vielzahl von Schlingenhaltern (132) umfassen, die durch Schlingen (131) an den Bewegungskanal gehängt sind.

3. Retraktor (100) nach Anspruch 2, **dadurch gekennzeichnet, dass** die Gewebeschieber (130) bewegliche Schieber (134) umfassen, die um eine Achse der Schlingenhalter (132) gedreht werden.

4. Retraktor (100) nach Anspruch 3, **dadurch gekennzeichnet, dass** die Gewebeschieber (130) Axialbewegungslager (133) umfassen, die die Drehbewegung zwischen den beweglichen Schiebern (134) und den Schlingenhaltern (132) bereitstellen.

5. Retraktor (100) nach Anspruch 3, **dadurch gekennzeichnet, dass** die Gewebeschieber (130) ein Gewebeschieberende (136) umfassen, das konfiguriert ist, um die Atriumwand des Herzens nach oben zu drücken und die Herzklappe sichtbar zu machen, verbunden mit der Bewegung der beweglichen Schieber (134).

6. Retraktor (100) nach Anspruch 4, **dadurch gekennzeichnet, dass** die Gewebeschieber (130) parallel zueinander angeordnete Zwischenrippen (135) aufweisen, die die Festigkeit der beweglichen Schieber (134) während eines Schiebens erhöhen Bewegung.

## Revendications

1. Un écarteur (100) comprenant un tube extérieur (110), sous la forme d'un segment de tore, avec une surface concave (112) qui fait face au centre d'un espace entouré, ladite surface concave comprenant au moins un canal de mouvement (111) formé sur ladite surface concave, un tube interne (120) sous la forme d'un segment de tore placé de manière mobile et télescopique dans une partie interne du tube extérieur (110), de sorte que ledit tube interne (120) et ledit tube extérieur (110) définissent ledit espace entouré, une pluralité de poussoirs de tissu (130) étant suspendus au canal de mouvement (111) et qui sont constitués de manière à pouvoir se déplacer le long du tube extérieur (110), ledit écarteur étant conçu pour être utilisé lors d'opérations à cœur ouvert pour rendre apparentes les valvules mitrale et tricuspide pendant l'opération chirurgicale, ledit écarteur étant **caractérisé en ce qu'il** comprend également:
- une pluralité de trous de réglage (121) formés sur ladite surface du tube interne (120) qui fait face au centre de l'espace entouré,
- des supports (122) fixés à chaque extrémité du tube extérieur (110) par des bagues de connexion (122.1), lesdits supports (122) étant fixés aux trous de réglage (121) dudit tube interne (120) par des broches (123).

2. Un écarteur (100) selon la revendication 1, **caractérisé en ce que** lesdits poussoirs de tissu (130) comprennent une pluralité de supports d'élingues (132) qui sont suspendus au canal de mouvement par des élingues (131).

3. Un écarteur (100) selon la revendication 2, **caractérisé en ce que** lesdits poussoirs de tissu (130) comprennent des poussoirs mobiles (134) qui tournent autour d'un axe des supports d'élingues (132).

4. Un écarteur (100) selon la revendication 3, **caractérisé en ce que** lesdits poussoirs de tissu (130) comprennent des paliers à mouvement axial (133) assurant le mouvement de rotation entre les poussoirs mobiles (134) et les supports d'élingue (132).

5. Un écarteur (100) selon la revendication 3, **caractérisé en ce que** lesdits poussoirs de tissu (130) comprennent une extrémité de poussoir de tissu (136), qui est configurée pour pousser la paroi de l'oreillette du coeur vers le haut et rend la valvule cardiaque apparente, alliée avec le mouvement des poussoirs mobiles (134).

6. Un écarteur (100) selon la revendication 4, **caractérisé en ce que** lesdits poussoirs de tissu (130) comprennent des nervures intermédiaires (135) placées parallèlement les unes aux autres, qui augmentent la résistance des poussoirs mobiles (134) pendant le mouvement de poussée.
